(19) Europäisches Patentamt
European Patent Office
Office européen des brevets

(11) **EP 3 716 957 B1**

(12) **EUROPEAN PATENT SPECIFICATION**

(45) Date of publication and mention
of the grant of the patent:
**19.07.2023 Bulletin 2023/29**

(21) Application number: **18829194.2**

(22) Date of filing: **26.11.2018**

(51) International Patent Classification (IPC):
**A61K 31/00** (2006.01) **A61K 31/56** (2006.01)
**A61K 31/568** (2006.01) **A61K 31/57** (2006.01)
**A61P 25/28** (2006.01)

(52) Cooperative Patent Classification (CPC):
**A61K 31/00; A61K 31/56; A61K 31/568;
A61K 31/57; A61P 25/28**

(86) International application number:
**PCT/CZ2018/050057**

(87) International publication number:
**WO 2019/101249 (31.05.2019 Gazette 2019/22)**

(54) **STEROIDAL COMPOUNDS FOR TREATMENT OF MENTAL AND NEUROLOGICAL DISORDERS**

STEROIDALE VERBINDUNGEN ZUR BEHANDLUNG VON MENTALEN UND NEUROLOGISCHEN ERKRANKUNGEN

COMPOSÉS STÉROÏDIENS POUR LE TRAITEMENT DE TROUBLES MENTAUX ET NEUROLOGIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.11.2017 CZ 20170757**

(43) Date of publication of application:
**07.10.2020 Bulletin 2020/41**

(73) Proprietors:
• **Ustav Organicke Chemie a Biochemie AV CR, v.v.i.**
**16610 Praha 6 (CZ)**
• **Fyziologicky ustav AV CR, v.v.i.**
**14000 Praha 4 (CZ)**

(72) Inventors:
• **KUDOVA, Eva**
**14800 Praha 4 (CZ)**
• **VYKLICKY, Ladislav**
**22168 Kamenice (CZ)**

(74) Representative: **Hartvichova, Katerina**
**HARBER IP s.r.o.**
**Dukelskych hrdinu 567/52**
**170 00 Praha 7 (CZ)**

(56) References cited:
**US-A1- 2004 204 490**

• **TYLER MARK PIERSON ET AL: "GRIN2A mutation and early-onset epileptic encephalopathy: personalized therapy with memantine", ANNALS OF CLINICAL AND TRANSLATIONAL NEUROLOGY, vol. 1, no. 3, 1 March 2014 (2014-03-01), pages 190-198, XP055558959, GB ISSN: 2328-9503, DOI: 10.1002/acn3.39**
• **L. ADDIS ET AL: "Epilepsy-associated GRIN2A mutations reduce NMDA receptor trafficking and agonist potency - molecular profiling and functional rescue", SCIENTIFIC REPORTS, vol. 7, no. 1, 27 February 2017 (2017-02-27), XP55559141, DOI: 10.1038/s41598-017-00115-w**
• **NAIL BURNASHEV ET AL: "NMDA receptor subunit mutations in neurodevelopmental disorders", CURRENT OPINION IN PHARMACOLOGY, vol. 20, 1 February 2015 (2015-02-01), pages 73-82, XP055559151, NL ISSN: 1471-4892, DOI: 10.1016/j.coph.2014.11.008**

- **HOGAN-CANN ADAM D ET AL: "Physiological Roles of Non-Neuronal NMDA Receptors", TRENDS IN PHARMACOLOGICAL SCIENCES, ELSEVIER, HAYWARTH, GB, vol. 37, no. 9, 21 June 2016 (2016-06-21), pages 750-767, XP029690020, ISSN: 0165-6147, DOI: 10.1016/J.TIPS.2016.05.012**
- **Zhou Qiang ET AL: "NMDA receptors in nervous system diseases", Neuropharmacology, vol. 74, 1 November 2013 (2013-11-01), pages 69-75, XP055972266, AMSTERDAM, NL ISSN: 0028-3908, DOI: 10.1016/j.neuropharm.2013.03.030**
- **Dobrek Lukasz ET AL: "Glutamate NMDA receptors in pathophysiology and pharmacotherapy of selected nervous system diseases", Postepy Hig Med Dosw, vol. 65, 7 June 2011 (2011-06-07), pages 338-346, XP055875848, Retrieved from the Internet: URL:https://phmd.pl/resources/html/article/details?id=51947&language=en>**

Remarks:

The complete document including Reference Table(s) and the Sequence Listing(s) can be downloaded from the EPO website

**Description**

**Field of Invention**

Steroidal compounds can potentiate NMDA receptor responses to rectify a defect induced by *de novo* mutations in the genes encoding its subunits. Such a defect leads to onset of major, mostly developmentally related, diseases, such as mental retardation, neurodevelopmental disorders in various areas - speech, motor activity, intellect, attention deficit with hyperactivity disorder, epilepsy, autism spectrum disorders (ASD), and schizophrenia.

**Background Art**

Increased use of new-generation DNA sequencing in neurological patients has led to supplementing of the knowledge of possible variants of mutated *N*-methyl-*D*-aspartate receptor (NMDAR) subtypes. They play an important role in normal brain development and their defects cause disorders such as epilepsy, speech disorders, motor disorders, learning disorders, autism spectrum disorders (ASD), attention deficit - hyperactivity disorder (ADHD), developmental disorders and schizophrenia. The functional results suggest that mutations in this region of the receptor have serious implications for receptor and neuronal functions, which may contribute to the occurrence of symptoms in patients as well as to damage to neurons.

The N-methyl-D-aspartate receptor (NMDAR) is a heteromeric ligand-gated ion channel permeable to $Ca^{2+}$ that is expressed in neurons and glia. Virtually all brain and spinal cord circuits rely on excitation evoked by transient activation of NMDARs by glutamate, giving rise to NMDAR excitatory postsynaptic currents (EPSCs) to regulate physiological functions. In addition, NMDAR signaling is implicated in various forms of synaptic plasticity (Dingledine et al., 1999; Lynch, 2004; Traynelis et al., 2010; Huganir and Nicoll, 2013).

NMDAR is composed of two obligatory subunits (GluN1) and two variable ones, which can be composed of either GluN2 (A-D) or GluN3 (A-B) (Traynelis et al., 2010). The GluN1 subunit is expressed throughout the central nervous system (CNS), whereas the four subtypes of GluN2 subunits (A-D) have differential temporal and spatial expression patterns (Akazawa et al., 1994; Monyer et al., 1994). Expression of the GluN2B subunit (encoded by *GRIN2B* gene) dominates during rapid cortical synaptogenesis in late embryogenesis and early postnatal development, and its expression level starts to decline after birth in most brain regions (Hall et al., 2007). Several lines of evidence indicate that GluN2B subunit plays an important role in brain development, neural circuit formation, differentiation, and synaptic plasticity (Cohen and Greenberg, 2008).

Recent advances in high-throughput DNA sequencing have allowed the examination of the prevalence of mutations in genes encoding NMDAR subunits in the afflicted individuals. While the gene encoding for the GluN2B subunit is most frequently *de novo* mutated *GRIN* gene in psychiatric and neurodevelopmental disorders, *de novo* mutations of *GRIN1* and *GRIN2A* genes encoding for GluN1 and GluN2A are less frequent and, interestingly, in *GRIN2C, GRIN3A,* and *GRIN3B,* only rare truncating mutations affecting both healthy individuals and patients suffering from neurodevelopmental disorders have been reported (Burnashev and Szepetowski, 2015; Hu et al., 2016).

Table 1: Subunits of NMDA receptor and encoding genes

| Receptor | Subunit | Gene | Chromosome |
|---|---|---|---|
| NMDA | GluN1 | GRIN 1 | 9q34.3 |
| | GluN2A | GRIN2A | 16p 13.2 |
| | GluN2B | GRIN2B | 12p12 |
| | GluN2C | GRIN2C | 17q24-q25 |
| | GluN2D | GRIN2D | 19q13.1qter |
| | GluN3A | GRIN3 A | 9q31.1 |
| | GluN3B | GRIN3B | 19p13.3 |

*GRIN2B* variants - *de novo* nonsynonymous mutations, missense, nonsense, frame shift, or splice site mutations - were identified in individuals with defined disorders such as intellectual disability (ID), developmental delay (DD), autism spectrum disorder (ASD), epileptic encephalopathy (EE), schizophrenia (SCZ), and to a lesser extent, attention deficit hyperactivity disorder (ADHD), and cerebral visual impairment (CVI) (Burnashev and Szepetowski, 2015; Hu et al., 2016).

Document US 2004/204490 discloses use of steroids for testing NMDA receptor response, but does not disclose any therapy.

### Disclosure of the Invention

The present invention relates a targeted rectification of defects caused by human *N*-methyl-*D*-aspartate receptor mutations with substances of steroid nature, useful for the treatment of disorders associated with genetically determined mutations of the said receptor subunits. Or, more precisely, to rectification of the dysfunction of the GluN2B subunits of the said receptor, caused by their encoding with the mutated GRIN2B gene. For simplicity, the term mutated subunits will also be used for such subunits. These mutations are the cause of serious, mostly developmentally related, diseases, such as mental retardation, neurodevelopmental disorders in various areas - speech, motor activity, intellect; attention deficit hyperactivity disorder (ADHD); epilepsy, autism spectrum disorders (ASD), and schizophrenia.

Recent research results have shown that mutation of genes encoding the NMDA receptor subunits (Table 1) may lead to various defects of the receptor function. It has been shown that the mutations described for the membrane part of the GluN2B subunit, having serious functional consequences, can be surprisingly rectified by the action of several steroidal agents.

Object of the invention is a steroidal compound selected from the group consisting of 20-oxo-pregn-5-ene-3β-yl sulfate, androst-5-ene-3β-yl hemisuccinate, 20-oxo-5β-pregnan-3β-yl butanoic acid and 20-oxo-5β-pregnan-3-ylidene-4'-but-2-enoic acid for use in the treatment of persons with a diagnosis including disorders of the intellect, delayed development, epilepsy, epileptic spasms, autism spectrum disorders, neurological developmental disorders in various areas - speech, motor activity, intellect; attention deficit hyperactivity disorder, schizophrenia.

The steroidal compound is selected from the group consisting of 20-oxo-pregn-5-ene-3β-yl sulfate, androst-5-ene-3β-yl hemisuccinate, 20-oxo-5β-pregnan-3β-yl butanoic acid, and 20-oxo-5β-pregnan-3-ylidene-4'-but-2-enoic acid.

It is an aspect of the invention that the mutation is a genetically determined mutation or *de novo* mutation of the membrane part of the GluN2B subunit.

It is an aspect of the invention that the mutation is a genetically determined or *de novo* mutation of the membrane part of the GluN2B subunit occurring in patients with a mental or neurological disorder.

It is a further aspect of the invention that the mutation of the GluN2B subunit is selected from the group consisting of mutations P553L, V558I, W607C, N615I, V618G, S628F, E657G, G820E, G820A, M824R and L825V, which were found in patients with mental or neurological disorder.

It is an aspect of the invention that the mutation of the GluN2B subunit is a L825V mutation in which the leucine at position 825 of the amino acid sequence of the GluN2B subunit is replaced by valine.

The aim of this invention was to elucidate the consequences of NMDA receptor mutations found in individuals diagnosed with neurodevelopmental disorders. Human NMDA receptors, consisting of hGluN1 and hGluN2B subunits (hGluN1/hGluN2B), have been shown to have a changed function when transmembrane domain (TMD) mutations in the GluN2B subunit were introduced. These mutations were identical to *de novo* mutations that have been described in patients with severe mental or neurological disease in which the causal relationship between the disease and the mutation is very likely. The list of the examined mutations is shown in Table 2, including phenotypic characterization and localization on the chromosome.

Table 2. Selected *de novo* GRIN2B variants and their phenotypic characteristics

| GluN2B mut. | Genotype | Phenotype | The age when it manifests | Literature |
|---|---|---|---|---|
| P553L | c.1658C>T | ID, hypotonia | Early postnatal | (de Ligt et al., 2012) |
| V558I | c. 1672G>A | ID | - | (Hamdan et al., 2014) (Lelieveld et al., 2016) |
| W607C | c. 1821G>T | ID, DD, dysmorphic features | - | (Yavarna et al., 2015) |
| N615I | c.1844A>T | WS, ID | 7 weeks | (Lemke et al., 2014) |
| V618G | c. 1853T>G | ID, WS, | 4 months | (Lemke et al., 2014) |
| S628F | c.1883C>T | ID, DD | - | (Platzer et al., 2017) |
| E657G | c. 1970A>G | ID, DD | - | (Platzer et al., 2017) |
| G820E | c. 2459G>A | ID, microcephaly | Early postnal | (Hamdan et al., 2014) |

(continued)

| GluN2B mut. | Genotype | Phenotype | The age when it manifests | Literature |
|---|---|---|---|---|
| G820A | c. 2459G>C | ID, DD, DDM, ES, GVL, ASD | - | (Platzer et al., 2017) |
| M824R | c. 2471T>G | ID, DD, microcephaly, clinical picture of Rett syndrome, Epi activity on EEG | 2 months | (Zhu et al., 2015) |
| L825V | c. 2473T>G | ASD | - | (Awadalla et al., 2010) (Swanger et al., 2016) |
| ID - intellectual disability; DD - developmental delay; WS - West syndrom; Epi - epilepsy and/or seizures, infantile spasms; ASD - autism spectrum disorders; DDM - Dyskinetic disturbance of movement; ES - epileptic spasms; GVL - generalized brain loss | | | | |

*De novo* mutations of the GRIN2B gene encoding the GluN2B subunit of the NMDA receptor are therefore associated with intellectual defect (ID), delayed development, epilepsy, epileptic spasms, and autism spectrum disorders (ASD). Even though these diseases are probably causally dependent on the mutation of the respective GRIN2B gene, implications for the NMDA receptor function have not yet been shown.

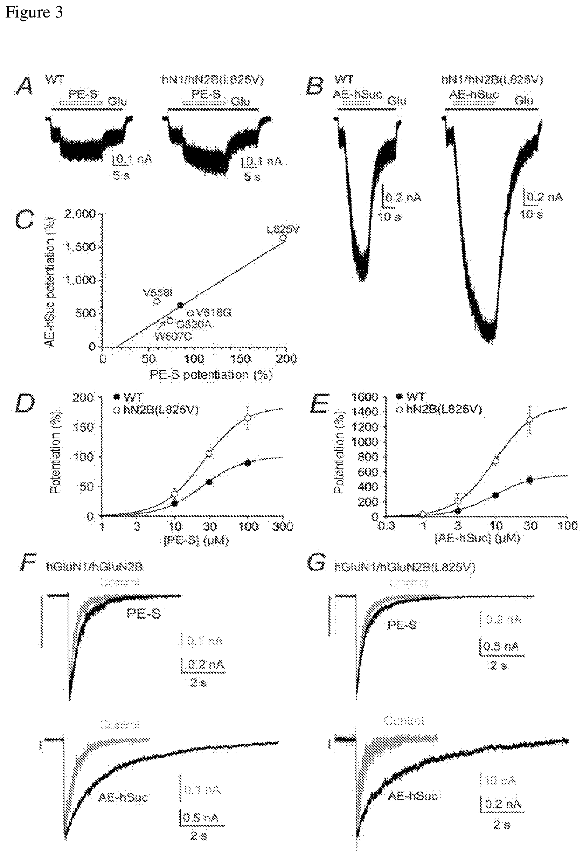

We have now found that the most significant functional changes in the receptor bearing a mutation in the GluN2B subunit relate to the probability of opening ($P_o$) (i.e. the relative ability of the NMDA receptor channels to be in the open state in the presence of saturating glutamate concentration). Based on our findings, the probability of opening has decreased at least 10-fold for different mutations in the GluN2B subunit. The tested steroidal compounds have shown the ability to rectify the defect induced by such mutation of human GluN2B subunits that decreased the probability of opening of the receptor channel. The analysis showed that the receptors containing the GluN2B subunit with the L825V mutation (i.e., GluN2B(L825V)) are markedly more potentiated by the tested steroids than the non-mutated (i.e. wild type) receptors (Figure 3A, Table 6), with an increase of up to 1600 %. In contrast, hGluN2B(V558I), hGluN2B(W607C), hGluN2B(V618G), and hGluN2B(G820A) mutations were potentiated similarly to wild type receptors (see Figure 3C and Table 6).

The ability of the steroidal compounds to rectify a defect related to a mutation of the GluN2B subunit, manifested in persons with mental or neurological disorder, was also tested *in vivo* in mice showing a change in social behavior. After the steroid administration, slight changes in the behavior of the animals were observed towards increased socialization.

However, these results (on recombinant receptors) represent a biologically extreme condition in which both alleles are *de novo* mutated, which is unlikely in practice. Therefore, in the case of mutation of one gene on one allele and in the presence of a healthy gene on the other allele, it can be assumed that the receptor will be formed according to the Mendelian genetics rules. The calculations show that if the mutated receptor were dominant, a 67% receptor response potentiation would be required to fully compensate for / rectify the mutation effect. In the case of a recessive mutant receptor variant, only moderate, about 20% potentiation would be required for complete compensation for / rectification of the mutation effect. The effect of the tested steroids exceeds these requirements by many times.

During the development, the neurons express different subunits and combinations thereof. In the case of the cortex, primarily a GluN2B subunit prevails, and, to a lesser extent, the GluN2A subunit. Their ratio is matched during the development. Then, in the adulthood, the GluN2A receptor subunit predominates. Therefore, it can be expected that even a lower degree of potentiation of the receptor will suffice to successfully rectify the defect.

## Description of Figures

### Figure 1
Localization of the mutations examined in the hGluN2B subunit. A: The NMDA receptor and its subunit composition. Protein sequence alignment of pre-M1, M1, M2, M3, and M4 regions across NMDA receptor subunits. The membrane segments are indicated with a horizontal line. **B:** Ribbon visualization of the GluN1/GluN2B receptor model (Karakas and Furukawa, 2014) (Lee et al., 2014). Selected amino acids that have been altered in human patients are highlighted. ABD = agonist binding domain, TMD = transmembrane domain, **C:** M1, M3, and M4 = transmembrane helices and M2 = re-entrant pore loop.

**Figure 2**

Mutations in the TMD of hGluN2B subunit affect the NMDA receptor surface expression. Quantitative colorimetric assay was used to determine the relative surface-to-total expression of the NMDA receptors hYFP-GluN1. COS-7 cells were transfected with either only the hYFP-GluN1 subunit or hYFP-GluN1 and WT (hGluN2B)/gene encoding the mutated hGluN2B subunit. Data show mean $\pm$ SEM; *P < 0.05 is the relative surface expression of hYFP-GluN1/hGluN2B (WT or mutant), statist. evaluation: one-way ANOVA (multiple comparisons versus WT (Dunnett's method)).

**Figure 3**

The hGluN1/hGluN2B(L825V) mutation is increasingly sensitive to steroid modulation, typical WT and hGluN2B/hGluN2B (L825V) receptor responses to steroid administration are shown here. PE-S (100 $\mu$mol/l) **(A)** and AE-hSuc (30 $\mu$mol/l) **(B)** potentiated hGluN2B/hGluN2B (WT) receptor responses induced by the rapid application of 1 $\mu$mol/l glutamate and measured in the presence of 30 $\mu$mol/l of glycine (potentiation by 110 % for PES and 612 % for AE-hSuc). Both steroids potentiated responses of hGluN2B/hGluN2B(L825V) receptors approximately three times more than those of WT receptors. **(C)** A graph of relative potentiation induced by PE-S (100 $\mu$mol/l) versus AE-hSuc induced potentiation (30 $\mu$mol/l) in WT and mutated receptors. Data was fit by linear regression (correlation coefficient r = 0.928; P = 0.00764). **(D,E)** Reactions of WT and hGluN2B/hGluN2B(L825V) receptors induced by short application (5 ms) of 1 mM glutamate (control) and glutamate in the presence of PE-S (100 $\mu$mol/l) or AE-hSuc (30 $\mu$mol/l). The vertical line at the top left indicates the amplitude of the WT response on the same scale as the amplitude recorded in the mutant receptors.

**Examples**

**Abbreviations**

NMDA: N-methyl-D- aspartate receptor
GluN1: subunit of the NMDA receptor
GluN2A: subunit of the NMDA receptor
hGluN2B: human subunit of the NMDA receptor
GluN2B: subunit of the NMDA receptor
YFP-hGluN1: human subunit of the NMDA receptor labeled with a yellow fluorescence protein
*GRIN2B:* gene encoding the GluN2B subunit
GluN2B(L825V): mutated subunit of GluN2B - leucine (L) at position 825 was replaced with valine (V)
preM1, M2, M3, M4: transmembrane helixes of NMDA receptor
PE-S: 20-oxo-pregn-5-en-3$\beta$-yl sulfate
AE-hSuc: androst-5-en-3$\beta$-yl hemisuccinate
PA-but: 20-oxo-5$\beta$-pregnan-3-ylidene-4'-but-2-enoic acid
BAPTA: (1,2-bis(*o*-aminophenoxy)ethane-N,N,N',N'-tetraacetic acid
HEPES: 4-(2-hydroxyethyl)-1-piperazineethanesulfonic acid
EDTA: ethylenediaminetetraacetic acid
ANOVA: statistic test
DNA: deoxyribonucleic acid
Opti-MEM: culture medium
ABD: agonist-binding domain
TMD: transmembrane domain
WT: wild type receptor (non-mutated)
ADHD: attention deficit hyperactivity disorder
ASD: autism spectrum disorders
NMDA EPSC: component of excitatory postsynaptic current induced by activation of NMDA receptors

**Transfection and maintenance of cells**

Human embryonic kidney-293T (HEK293T) cells were used for transfection of human genes encoding for NMDA receptor subunits: hGluN1 (GenBank, accession number NP_015566) and hGluN2B (GenBank, accession number NP_000825). Equal amounts (0.25 mg) of DNA encoding for GluN1 and GluN2B were mixed with 0.9 $\mu$l Matra-A Reagent (IBA, Göttingen, Germany) and added to confluent HEK293T cells. After trypsinization, the cells were resuspended in Opti-MEM I (ThermoFisher Scientific, Paisley, UK; Cat. # 31985062) containing 1% fetal bovine serum supplemented with 15 mmol.l$^{-1}$ MgCl$_2$, 1 mmol.l$^{-1}$ D,L-2-amino-5-phosphonopentanoic acid (D,L-AP5), and 1 μmol.l$^{-1}$ ketamine.

Mutations in the GluN2B subunit were made using the QuickChange Site-Directed Mutagenesis Kit (Agilent Technologies, Santa Clara, CA, USA) according to the manufacturer's instructions. Mutated cDNA was transformed into ultra-competent XL1Gold *E.coli* cells, multiplied, positive clones were selected, and the isolated plasmide DNA was verified by sequencing (SEQme s.r.o., Dobris, Czechia, or GATC Biotech AG, Constance, Germany). All mutations were verified by DNA sequencing. Transfected HEK293T cells were identified by GFP epifluorescence. The amino acids are numbered on the basis of the full-length protein, including the signal peptide.

**Electrophysiological recording**

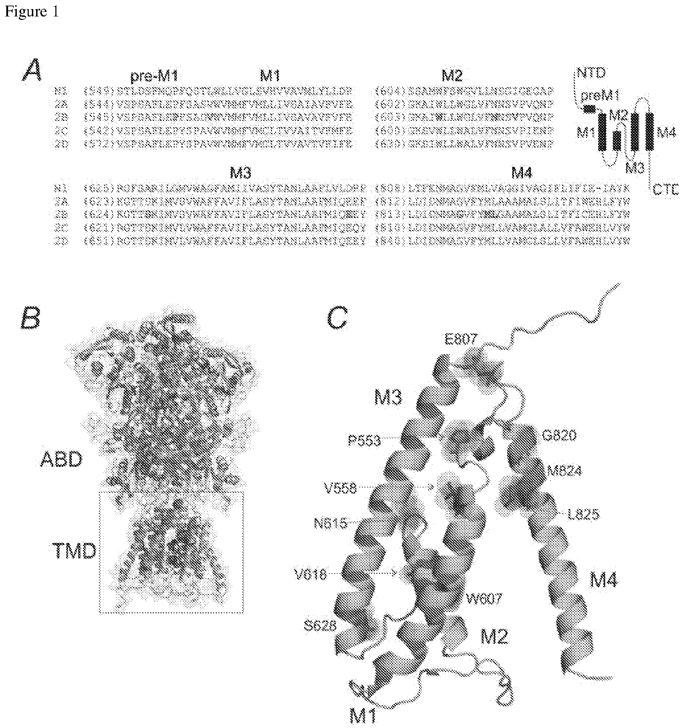

Experiments were performed 12-36 h after the end of transfection on cultured HEK293T cells transfected with vectors containing hGluN1/hGluN2B/GFP. Whole-cell voltage clamp current recordings were performed at room temperature (21-25°C) at a holding potential of -60 mV using a patch-clamp amplifier (Axopatch 200B; Molecular Devices, Sunnyvale, CA, USA) after a capacitance and series resistance (<10 MΩ) compensation of 80-90%. Data were collected (low-pass filtered at 2 kHz and sampled at 10 kHz) and analyzed using pClamp 10 (Molecular Devices). Patch pipettes (3-5 MΩ) were filled with an intracellular solution (ICS) containing (in mmol.l$^{-1}$): 120 gluconic acid, 15 CsCl, 10 BAPTA, 10 HEPES, 3 MgCl$_2$, 1 CaCl$_2$, and 2 ATP-Mg salt (pH-adjusted to 7.2 with CsOH) and extracellular solution (ECS) contained the following (in mmol.l$^{-1}$): 160 NaCl, 2.5 KCl, 10 HEPES, 10 glucose, 0.2 EDTA, and 0.7 CaCl$_2$ (pH-adjusted to 7.3 with NaOH). Glycine (30 μM), an NMDA receptor co-agonist, was present in the control and test solutions. The tested steroid solutions were made from freshly prepared 20 mmol.l$^{-1}$ 20-oxopregn-5en-3β-yl sulfate (PE-S; pregnenolone sulfate), 5 mmol.l$^{-1}$ androst-5-en-3β-yl hemisuccinate (AND-hSuc), or 10 mmol.l$^{-1}$ 20-oxo-5β-pregnan-3-yliden-4'-but-2-enoic acid stock in dimethyl sulfoxide (DMSO). The final dilution to ECS was made at 50 °C and followed by 1 min sonication (SONOREX DIGITEC DT 100/H, BADELIN electronic, Berlin, Germany). The same concentration of DMSO was added to all extracellular solutions. Control and test solution applications were made with a microprocessor-controlled multibarrel fast-perfusion system. The solution exchange rate was ~15 ms. The following mutations of the gene encoding for hGluN2B subunit (GRIN2B) were tested: P553L; V558I; W607C; N615I; V618G; S628F; E657G; G820E; G820A; M824R; L825V. These mutations are located in the preM1, M2, M3, and M4 transmembrane domains of the receptor (Fig. 1).

It was found that responses induced by a saturating concentration of glutamate in HEK293T cells transfected by human wild type (WT) hGluN1/hGluN2B receptors had value of 66 pA/pF (current response normalized with respect to the cell capacitance). In mutated receptors (hGluN2B(V5581) and (hGluN2B(L825V) the responses were of similar amplitude to the WT, reduced or did not respond to the glutamate application (Table 3).

Table 3. Relative responses induced in HEK293T cells transfected by WT hGluN1 and WT or mutated hGluN2B receptors

| Receptors (hGluN1/ ) | Current response (pA/pF) |
|---|---|
| hGluN2B | 66 ± 15 (23) |
| hGluN2B(P553L) | NR (5) |
| hGluN2B(V558I) | 30 ± 11 (12)[†]; P = 0.068[‡] |
| hGluN2B(W607C) | 6.1 ± 1.6 (11)[†]; P = <0.001[‡] |
| hGluN2B(N615I) | 17 ± 5 (9)[†]; P = 0.021[‡] |
| hGluN2B(V618G) | 2.7 ± 1.0 (11)[†]; P = <0.001[‡] |
| hGluN2B(S628F) | NR (8) |
| hGluN2B(E657G) | 0.7 ± 0.2 (11)[†]; P = <0.001[‡] |
| hGluN2B(G820E) | NR (5) |
| hGluN2B(G820A) | 1.8 ± 0.6 (7)[†]; P = 0.002[‡] |
| hGluN2B(M824R) | NR (6) |
| hGluN2B(L825V) | 67 ± 8 (17)[†]; P = 0.198[‡] |

Summary of current amplitudes induced by 1 mmol.l$^{-1}$ glutamate and normalized with respect to the HEK293T cell capacitance.

The data are expressed as mean $\pm$ SEM

(*n*) is the number of cells recorded from, NR = non-responding
† Kruskal-Wallis One Way Analysis of Variance on Ranks P = <0.001
‡ Mann-Whitney Rank Sum Test, P indicates significance level compared to the WT

A detailed analysis of those receptors that had low sensitivity to glutamate was focused on four possible problems that can be combined: reduced surface expression of the receptor, change in desensitization, decreased probability of opening, and altered sensitivity to glutamate or glycine - see Table 4. The most significant changes were found in the probability of opening ($P_o$) (i.e. in the relative ability of the NMDA receptor channels to be in the presence of saturating glutamate concentration in the open state). The probability of opening of wild type hGluN1/hGluN2B receptors is approximately 10%. In the case of differently mutated receptors, the probability of opening decreased approximately 10-fold (see Table 5).

Table 4. Summary of glutamate and glycine activity for mutations in TMD

| Receptor hGluN1/ | Glutamate EC$_{50}$ $\mu$mol.l$^{-1}$ (*n*) *h* | Glycine EC$_{50}$ $\mu$mol.l$^{-1}$ (*n*) *h* |
|---|---|---|
| hGluN2B | 1.6 $\pm$ 0.1 (25)[#] | 0.23 $\pm$ 0.02 (19)[#] |
| hGluN2B (V558I) | 1.6 $\pm$ 0.3 (6)[§] | 0.22 $\pm$ 0.05 (5)[‡] |
| hGluN2B(W607C) | 5.1 $\pm$ 0.4 (5)[# **] | 0.43 $\pm$ 0.05 (12)[# **] |
| hGluN2B(N615I) | 2.7 $\pm$ 0.2 (5)[# **] | 0.31 $\pm$ 0.02 (5)[‡] |
| hGluN2B(V618G) | 1.8 $\pm$ 0.2 (8)[§] | 0.27 $\pm$ 0.02 (7)[‡] |
| hGluN2B(E657G) | 0.5 $\pm$ 0.1 (5)[# **] | 0.39 $\pm$ 0.08 (5)[‡] |
| hGluN2B(G820A) | 1.5 $\pm$ 0.3 (4)[§] | 0.20 $\pm$ 0.09 (3)[‡] |
| hGluN2B(L825V) | 1.6 $\pm$ 0.2 (12)[§] | 0.16 $\pm$ 0.01 (5)[‡] |

§ Ratio of responses to 1 mmol.l$^{-1}$ and 3 mmol.l$^{-1}$ glutamate was used to calculate EC$_{50}$ (see Eq. 1; *h* was fixed at 1.4).

$$I = \frac{1}{1 + \left(\dfrac{EC_{50}}{[agonist]}\right)^{h}} \qquad\qquad \text{Eq. 1}$$

‡ Ratio of responses to 30 mmol.l$^{-1}$ and 0.3 mmol.l$^{-1}$ glycine was used to calculate EC50 (see Eq. 1; *h* was fixed at 1.4).
# EC$_{50}$ was determined from a fit to the logistic equation (see Eq. 1) of currents induced by 0.3, 1, 3, 10, 30 mmol.l$^{-1}$ glutamate in the continuous presence of 30 mmol.l$^{-1}$ glycine or 0.1, 0.3, 1, 3, 10, 30 mmol.l$^{-1}$ glycine applied together with 1 mmol.l$^{-1}$ glutamate.

The data are expressed as mean $\pm$ SEM.

*n* is the number of cells recorded from.
**p < 0.001; statistical tests on potency were performed on logEC$_{50}$ and logHill compared to the corresponding WT; one-way ANOVA, Tukey post-hoc Dunnett's method

Table 5. Mutations in the TMD alter receptor desensitization and probability of channel opening (Po)

| Receptor (hGluN1/) | Desensitization (%), (*n*) | P$_0$ (%), (*n*) |
|---|---|---|
| hGluN2B | 16.1 $\pm$ 2.2 (10) | 10.48 $\pm$1.30 (10) |
| hGluN2B (V558I) | 72.9 $\pm$ 3.4 (5); P = <0.001 | 0.66 $\pm$ 0.11 (5); P = 0.003 |
| hGluN2B(W607C) | 23.5 $\pm$ 003 (4); P = 0.088 | 0.89 $\pm$ 0.25 (4); P = 0.006 |

(continued)

| Receptor (hGluN1/) | Desensitization (%), (n) | $P_0$ (%), (n) |
|---|---|---|
| hGluN2B(V618G) | 4.1 ± 2.2 (4); P = 0.008 | 0.49 ± 0.16 (4); P = 0.006 |
| hGluN2B(L825V) | 19.8 ± 3.8 (6); P = 0.357 | 1.47 ± 1.8 (10); P = 0.001 |

The data are expressed as mean ± SEM, (n) is the number of cells recorded from.

Statistical analysis: Kruskal-Wallis one-way ANOVA on Ranks, P < 0.001; subsequent Mann-Whitney Rank Sum Test or unpaired t-test was used to assess the significance compared to the WT, P indicates significance compared to WT.

Three selected steroids - 20-oxo-pregn-5-ene-3β-yl sulfate (PES), androst-5-ene-3β-yl hemisuccinate (AE) and 20-oxo-5β-pregnan-3-yliden-4'-but-2-enoic acid, which are known to potentiate rat NMDA receptor responses were used to affect the receptors being tested. The aim was to determine whether and to what extent the steroids would be able to rectify the mutation-induced defect of human GluN2B. Analysis revealed that GluN2B containing L825V-containing receptors (i.e. GluN2B (L825V)) were significantly more potentiated by PE-S (100 μmol/l) than wild type (non mutated) receptors (Figure 3A, Table 6). A similar, more pronounced effect on mutant receptors was also found for AE-hSuc (30 μmol/l). GluN2B (L825V) were potentiated by AE-hSuc by 1647 %. Receptors containing hGluN2B (V558I), hGluN2B (W607C), hGluN2B (V618G) and hGluN2B (G820A) were potentiated similarly to wild type receptors (see Figure 3C and Table 6). The analysis of concentration dependency for steroid induced potentiation has shown that the augmented potentiation of GluN1/GluN2B(L825V) is attributable to the increase in steroid efficacy rather than potency (see Figure 3D).

Table 6. Summary of potentiating effects of steroids on mutated receptors, tonically activated by low concentration of glutamate.

| Receptor (hGluN1/) | PE-S (100 μmol.l⁻¹) (%) | AE-hSuc (30 μmol.l⁻¹) (%) | PA-But (15 μmol.l⁻¹) (%) |
|---|---|---|---|
| hGluN2B | 85 ± 4 (31) | 628 ± 71 (19) | 212 ± 19 (22) |
| hGluN2B(V558I) | 59 ± 8 (5)[†]; P = 0.117[‡] | 685 ± 67 (6)[††]; P = 0.448[‡] | -9 ± 5 (4)[††]; P = <0.001[‡] |
| hGluN2B(W607C) | 73 ± 16 (8)[†]; P = 0.412[‡] | 406 ± 49 (8)[††]; P = 0.489[‡] | 160 ± 24 (3)[††]; P = 0.885[‡] |
| hGluN2B(V618G) | 96 ± 10 (8)[†]; P = 0.542[‡] | 509 ± 61 (5)[††]; P = 0.966[‡] | 67 ± 12 (3)[††]; P = 0.002[‡] |
| hGluN2B(G820A) | 74 ± 21 (5)[†]; P = 0.271[‡] | 411 ± 138 (3)[††]; P = 0.498[‡] | 7 ± 3 (5) [††]; P = <0.001[‡] |
| hGluN2B(L825V) | 197 ± 36 (9)[†]; P = <0.001[‡] | 1647 ± 425 (7)[††]; P = <0.001[‡] | 577 ± 78 (10)[††]; P = <0.001[‡] |

The data are expressed as mean ± SEM, (n) is the number of cells recorded from. [†/††]Kruskal-Wallis one-way ANOVA on Ranks, P < 0.001; subsequent [‡]Mann-Whitney Rank Sum Test or unpaired t-test was used to assess the significance compared to the WT, P indicates significance compared to WT.

Investigation of the effect of steroids on a short-term, approximately 5 ms administration of 1 mmol.l⁻¹ of glutamate (this mode of administration mimics the activation of NMDA receptors during excitatory synaptic transmission) showed that the amplitude of these responses was increased (see Figure 3F, 3G and Tables 7 and 8) and that the deactivation slows down (Figure 3F, 3G and Tables 7 and 8).

Table 7. Summary of pharmacological properties of "synaptic-like" responses to the wild type hGluN1/hGluN2B receptor.

| | Control | PE-S (100 μmol.l⁻¹) | Control | AE-hSuc (30 μmol.l⁻¹) |
|---|---|---|---|---|
| Potentiation (%) | - | 98.7 ± 11.5 (5) | - | 924.5 ± 172.7 (5) |
| τ (ms) | 307 ± 14 (5)[†] | 499 ± 20 (5)[‡] *P = <0.001 | 370 ± 35 (5)[†] | 5961 ± 542 (5)[††] *P = <0.001 |

(continued)

| | Control | PE-S (100 μmol.l⁻¹) | Control | AE-hSuc (30 μmol.l⁻¹) |
|---|---|---|---|---|
| Deceleration | - | 1.63 ± 0.06 (5) | - | 16.36 ± 1.44 (5)†† |
| Charge transfer | - | 3.26 ± 0.30 (5) | - | 161.36 ± 20.28 (5) |

Table 8. Summary of the pharmacological properties of the "synaptic-like" response of the mutated hGluN1/hGluN2B (L825V) receptor.

| | Control | PE-S (100 μmol.l⁻¹) | Control | AE-hSuc (30 μmol.l⁻¹) |
|---|---|---|---|---|
| Potentiation (%) | - | 140.8 ± 10.8 (5) | - | 2323.1 ± 120.7 (5) |
| $\tau$ (ms) | 323 ± 9 (5)† | 507 ± 28 (5)‡ *P = <0.001 | 335 ± 29 (5)† | 2467 ± 168 (5)†† *P = <0.001 |
| Deceleration | - | 1.57 ± 0.05 (5) | - | 7.19 ± 0.59 (5)†† |
| Charge transfer | - | 3.79 ± 0.25 (5) | - | 179.65 ± 9.11 (5) |

In both tables (7 and 8) the data are expressed as mean ± SEM, (*n*) is the number of cells recorded from.

* Paired t-test, P indicates significance compared to control
† $\tau$ determined from the single exponential fit
‡ $\tau_w$ determined from the double exponential fit
Deceleration is defined as $\tau_{steroid}/\tau_{control}$
Charge transfer is defined as $\tau \times \left( \dfrac{potentiation}{100} + 1 \right)$

## In vivo experiments

In *in vivo* experiments, the effect of the steroid 20-oxo-5β-pregnan-3-ylidene-4'-but-2-enoic acid on C57BL/6 mice (control) and BALB/c mice was compared. BALB/c mice show a higher rate of anxiety behavior and lower socialization, which is considered to be a certain equivalent of autistic manifestations in humans.
After intraperitoneal administration of doses of 1 and 10 mg/kg of the above-mentioned steroid dissolved in cyclodextrine, the animals showed a greater interest in the second mouse cage than in an empty cage in the three-chamber social behavior test, which can be understood as a sign of a reduction in social behavioral defects.

## Industrial applicability

Affecting the mutation on the N-methyl-D-aspartate receptor subunit can provide an effective treatment for developmentally related diseases selected from mental retardation, neurodevelopmental disorders in various areas - speech, motor activity, intellect, attention deficit hyperactivity disorder; epilepsy, autism spectrum disorders (ASD), and schizophrenia.

## Literature

Akazawa C, Shigemoto R, Bessho Y, Nakanishi S, Mizuno N (1994) Differential expression of five N-methyl-D-aspartate receptor subunit mRNAs in the cerebellum of developing and adult rats. J Comp Neurol 347:150-160.
Awadalla P et al. (2010) Direct measure of the de novo mutation rate in autism and schizophrenia cohorts. American journal of human genetics 87:316-324.
Burnashev N, Szepetowski P (2015) NMDA receptor subunit mutations in neurodevelopmental disorders. Curr Opin Pharmacol 20:73-82.
Cohen S, Greenberg ME (2008) Communication between the synapse and the nucleus in neuronal development, plasticity, and disease. Annu Rev Cell Dev Biol 24:183-209.

de Ligt J, Willemsen MH, van Bon BW, Kleefstra T, Yntema HG, Kroes T, Vulto-van Silfhout AT, Koolen DA, de Vries P, Gilissen C, del Rosario M, Hoischen A, Scheffer H, de Vries BB, Brunner HG, Veltman JA, Vissers LE (2012) Diagnostic exome sequencing in persons with severe intellectual disability. N Engl J Med 367:1921-1929.

Dingledine R, Borges K, Bowie D, Traynelis SF (1999) The glutamate receptor ion channels. Pharmacological reviews 51:7-61.

Hall BJ, Ripley B, Ghosh A (2007) NR2B signaling regulates the development of synaptic AMPA receptor current. The Journal of neuroscience : the official journal of the Society for Neuroscience 27:13446-13456.

Hamdan FF, Srour M, Capo-Chichi JM, Daoud H, Nassif C, Patry L, Massicotte C, Ambalavanan A, Spiegelman D, Diallo O, Henrion E, Dionne-Laporte A, Fougerat A, Pshezhetsky AV, Venkateswaran S, Rouleau GA, Michaud JL (2014) De novo mutations in moderate or severe intellectual disability. PLoS genetics 10:e1004772.

Hu C, Chen W, Myers SJ, Yuan H, Traynelis SF (2016) Human GRIN2B variants in neurodevelopmental disorders. Journal of pharmacological sciences 132:115-121. Huganir RL, Nicoll RA (2013) AMPARs and synaptic plasticity: the last 25 years. Neuron 80:704-717.

Karakas E, Furukawa H (2014) Crystal structure of a heterotetrameric NMDA receptor ion channel. Science 344:992-997.

Lee CH, Lu W, Michel JC, Goehring A, Du J, Song X, Gouaux E (2014) NMDA receptor structures reveal subunit arrangement and pore architecture. Nature 511:191-197.

Lelieveld SH et al. (2016) Meta-analysis of 2,104 trios provides support for 10 new genes for intellectual disability. Nat Neurosci 19:1194-1196.

Lemke JR, Hendrickx R, Geider K, Laube B, Schwake M, Harvey RJ, James VM, Pepler A, Steiner I, Hortnagel K, Neidhardt J, Ruf S, Wolff M, Bartholdi D, Caraballo R, Platzer K, Suls A, De Jonghe P, Biskup S, Weckhuysen S (2014) GRIN2B mutations in West syndrome and intellectual disability with focal epilepsy. Annals of neurology 75:147-154.

Lynch MA (2004) Long-term potentiation and memory. Physiol Rev 84:87-136.

Monyer H, Burnashev N, Laurie DJ, Sakmann B, Seeburg PH (1994) Developmental and regional expression in the rat brain and functional properties of four NMDA receptors. Neuron 12:529-540.

Platzer K et al. (2017) GRIN2B encephalopathy: novel findings on phenotype, variant clustering, functional consequences and treatment aspects. J Med Genet 54:460-470.

Swanger SA, Chen W, Wells G, Burger PB, Tankovic A, Bhattacharya S, Strong KL, Hu C, Kusumoto H, Zhang J, Adams DR, Millichap JJ, Petrovski S, Traynelis SF, Yuan H (2016) Mechanistic Insight into NMDA Receptor Dysregulation by Rare Variants in the GluN2A and GluN2B Agonist Binding Domains. American journal of human genetics 99:1261-1280.

Traynelis SF, Wollmuth LP, McBain CJ, Menniti FS, Vance KM, Ogden KK, Hansen KB, Yuan H, Myers SJ, Dingledine R, Sibley D (2010) Glutamate receptor ion channels: structure, regulation, and function. Pharmacological reviews 62:405-496.

Yavarna T, Al-Dewik N, Al-Mureikhi M, Ali R, Al-Mesaifri F, Mahmoud L, Shahbeck N, Lakhani S, AlMulla M, Nawaz Z, Vitazka P, Alkuraya FS, Ben-Omran T (2015) High diagnostic yield of clinical exome sequencing in Middle Eastern patients with Mendelian disorders. Hum Genet 134:967-980.

Zhu X et al. (2015) Whole-exome sequencing in undiagnosed genetic diseases: interpreting 119 trios. Genet Med 17:774-781.

**Claims**

1. A steroidal compound selected from the group consisting of 20-oxo-pregn-5-ene-β-yl sulfate, androst-5-ene-3β-yl hemisuccinate, 20-oxo-5β-pregnane-3β-yl butanoic acid and 20-oxo-5β-pregnan-3-ylidene-4'-but-2-enoic acid for use in the treatment of patients suffering from a mental or neurological disorder caused by a mutation occurring in the membrane region of a human N-methyl-D-aspartate receptor subunit, wherein said disorder is selected from intellectual disability, developmental delay, epilepsy, epileptic spasms, autism spectrum disorders, neurological developmental disorders in speech, motor activity or intellect, attention deficit hyperactivity disorder and schizophrenia.

2. The steroidal compound for use according to claim 1, wherein the mutation is a genetically determined mutation or *de novo* mutation of the membrane region of GluN2B subunit.

3. The steroidal compound for use according to claim 2, wherein the mutation of the GluN2B subunit is selected from the group comprising the mutations P553L, V5581, W607C, N615I, V618G, S628F, E657G, G820E, G820A, M824R and L825V.

4. The steroidal compound for use according to claim 3, wherein the mutation of the GluN2B subunit is L825V mutation, in which leucine in position 825 of the amino acid sequence of the GluN2B subunit is replaced by valine.

**Patentansprüche**

1. Eine Steroidverbindung, ausgewählt aus der Gruppe bestehend aus 20-Oxo-Pregnan-5-en-3β-yl-Sulfat, Androst-5-en-3β-yl-Hemisuccinat, 20-Oxo-5β-Pregnan-3β-ylbutansäure und 20-Oxo-5β-Pregnan-3-yliden-4'-but-2-ensäure, zur Verwendung bei der Behandlung von Patienten, die an einer geistigen oder neurologischen Störung leiden, die durch eine Mutation verursacht wird, die in der Membranregion einer humanen N-Methyl-D-Aspartat-Rezeptoruntereinheit auftritt, wobei die Störung ausgewählt ist aus geistiger Behinderung, Entwicklungsverzögerung, Epilepsie, epileptischen Krämpfen, Autismus-Spektrum-Störungen, neurologischen Entwicklungsstörungen in Sprache, Motorik oder Intellekt, Aufmerksamkeitsdefizit-Hyperaktivitätsstörung und Schizophrenie.

2. Eine Steroidverbindung zur Verwendung nach Anspruch 1, wobei die Mutation eine genetisch bestimmte Mutation oder eine *de novo*-Mutation der Membranregion der GluN2B-Untereinheit ist.

3. Eine Steroidverbindung zur Verwendung nach Anspruch 2, wobei die Mutation der GluN2B-Untereinheit ausgewählt ist aus der Gruppe, die die Mutationen P553L, V558I, W607C, N615I, V618G, S628F, E657G, G820E, G820A, M824R und L825V umfasst.

4. Eine Steroidverbindung zur Verwendung nach Anspruch 3, wobei die Mutation der GluN2B-Untereinheit eine L825V-Mutation ist, bei dem das Leucin in Position 825 der Aminosäuresequenz der GluN2B-Untereinheit durch Valin ersetzt ist.

**Revendications**

1. Composé stéroïdien choisi dans le groupe constitué par le 20-oxo-pregn-5-ène-3β-yl sulfate, l'androst-5-ène-3β-yl hémisuccinate, l'acide 20-oxo-5β-pregnane-3β-yl butanoïque et l'acide 20-oxo-5β-pregnane-3-ylidène-4'-but-2-énoïque pour une utilisation dans le traitement de patients souffrant d'un trouble mental ou neurologique causé par une mutation survenant dans la région membranaire d'une sous-unité du récepteur N-méthyl-D-aspartate humain, dans lequel ledit trouble est choisi parmi la déficience intellectuelle, le retard de développement, l'épilepsie, les spasmes épileptiques, les troubles du spectre autistique, les troubles du développement neurologique de la parole, de l'activité motrice ou de l'intellect, le trouble d'hyperactivité avec déficit de l'attention et la schizophrénie.

2. Composé stéroïdien pour utilisation selon la revendication 1, où la mutation est une mutation génétiquement déterminée ou une mutation de novo de la région membranaire de la sous-unité GluN2B.

3. Composé stéroïdien pour utilisation selon la revendication 2, où la mutation de la sous-unité GluN2B est choisie dans le groupe comprenant les mutations P553L, V558I, W607C, N615I, V618G, S628F, E657G, G820E, G820A, M824R et L825V.

4. Composé stéroïdien pour utilisation selon la revendication 3, où la mutation de la sous-unité GluN2B est la mutation L825V, dans laquelle la leucine en position 825 de la séquence d'acides aminés de la sous-unité GluN2B est remplacée par la valine.

Figure 1

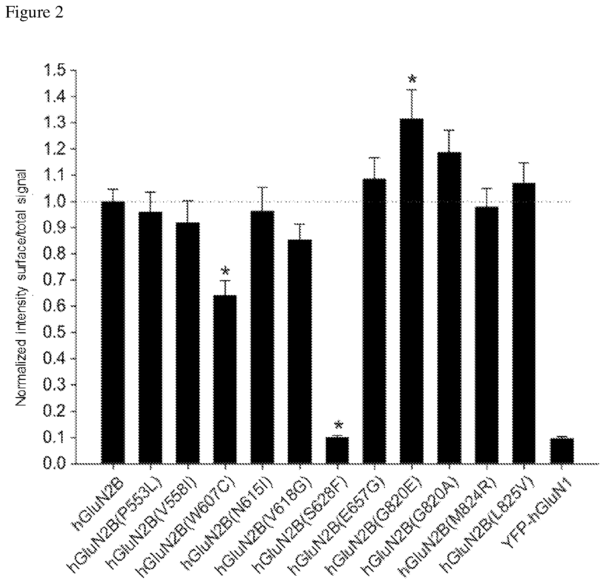

Figure 2

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004204490 A

**Non-patent literature cited in the description**

- **AKAZAWA C ; SHIGEMOTO R ; BESSHO Y ; NAKANISHI S ; MIZUNO N.** Differential expression of five N-methyl-D-aspartate receptor subunit mRNAs in the cerebellum of developing and adult rats. *J Comp Neurol,* 1994, vol. 347, 150-160
- **AWADALLA P et al.** Direct measure of the de novo mutation rate in autism and schizophrenia cohorts. *American journal of human genetics,* 2010, vol. 87, 316-324
- **BURNASHEV N ; SZEPETOWSKI P.** NMDA receptor subunit mutations in neurodevelopmental disorders. *Curr Opin Pharmacol,* 2015, vol. 20, 73-82
- **COHEN S ; GREENBERG ME.** Communication between the synapse and the nucleus in neuronal development, plasticity, and disease. *Annu Rev Cell Dev Biol,* 2008, vol. 24, 183-209
- **LIGT J ; WILLEMSEN MH ; VAN BON BW ; KLEEFSTRA T ; YNTEMA HG ; KROES T ; VULTO-VAN SILFHOUT AT ; KOOLEN DA ; DE VRIES P ; GILISSEN C.** Diagnostic exome sequencing in persons with severe intellectual disability. *N Engl J Med,* 2012, vol. 367, 1921-1929
- **DINGLEDINE R ; BORGES K ; BOWIE D ; TRAYNELIS SF.** The glutamate receptor ion channels. *Pharmacological reviews,* 1999, vol. 51, 7-61
- **HALL BJ ; RIPLEY B ; GHOSH A.** NR2B signaling regulates the development of synaptic AMPA receptor current. *The Journal of neuroscience : the official journal of the Society for Neuroscience,* 2007, vol. 27, 13446-13456
- **HAMDAN FF ; SROUR M ; CAPO-CHICHI JM ; DAOUD H ; NASSIF C ; PATRY L ; MASSICOTTE C ; AMBALAVANAN A ; SPIEGELMAN D ; DIALLO O.** De novo mutations in moderate or severe intellectual disability. *PLoS genetics,* 2014, vol. 10, e1004772
- **HU C ; CHEN W ; MYERS SJ ; YUAN H ; TRAYNELIS SF.** Human GRIN2B variants in neurodevelopmental disorders. *Journal of pharmacological sciences,* 2016, vol. 132, 115-121
- **HUGANIR RL ; NICOLL RA.** AMPARs and synaptic plasticity: the last 25 years. *Neuron,* 2013, vol. 80, 704-717
- **KARAKAS E ; FURUKAWA H.** Crystal structure of a heterotetrameric NMDA receptor ion channel. *Science,* 2014, vol. 344, 992-997
- **LEE CH ; LU W ; MICHEL JC ; GOEHRING A ; DU J ; SONG X ; GOUAUX E.** NMDA receptor structures reveal subunit arrangement and pore architecture. *Nature,* 2014, vol. 511, 191-197
- **LELIEVELD SH et al.** Meta-analysis of 2,104 trios provides support for 10 new genes for intellectual disability. *Nat Neurosci,* 2016, vol. 19, 1194-1196
- **LEMKE JR ; HENDRICKX R ; GEIDER K ; LAUBE B ; SCHWAKE M ; HARVEY RJ ; JAMES VM ; PEPLER A ; STEINER I ; HORTNAGEL K.** GRIN2B mutations in West syndrome and intellectual disability with focal epilepsy. *Annals of neurology,* 2014, vol. 75, 147-154
- **LYNCH MA.** Long-term potentiation and memory. *Physiol Rev,* 2004, vol. 84, 87-136
- **MONYER H ; BURNASHEV N ; LAURIE DJ ; SAKMANN B ; SEEBURG PH.** Developmental and regional expression in the rat brain and functional properties of four NMDA receptors. *Neuron,* 1994, vol. 12, 529-540
- **PLATZER K et al.** GRIN2B encephalopathy: novel findings on phenotype, variant clustering, functional consequences and treatment aspects. *J Med Genet,* 2017, vol. 54, 460-470
- **SWANGER SA ; CHEN W ; WELLS G ; BURGER PB ; TANKOVIC A ; BHATTACHARYA S ; STRONG KL ; HU C ; KUSUMOTO H ; ZHANG J.** Mechanistic Insight into NMDA Receptor Dysregulation by Rare Variants in the GluN2A and GluN2B Agonist Binding Domains. *American journal of human genetics,* 2016, vol. 99, 1261-1280
- **TRAYNELIS SF ; WOLLMUTH LP ; MCBAIN CJ ; MENNITI FS ; VANCE KM ; OGDEN KK ; HANSEN KB ; YUAN H ; MYERS SJ ; DINGLEDINE R.** Glutamate receptor ion channels: structure, regulation, and function. *Pharmacological reviews,* 2010, vol. 62, 405-496

- **YAVARNA T ; AL-DEWIK N ; AL-MUREIKHI M ; ALI R ; AL-MESAIFRI F ; MAHMOUD L ; SHAH-BECK N ; LAKHANI S ; ALMULLA M ; NAWAZ Z.** High diagnostic yield of clinical exome sequencing in Middle Eastern patients with Mendelian disorders. *Hum Genet,* 2015, vol. 134, 967-980

- **ZHU X et al.** Whole-exome sequencing in undiagnosed genetic diseases: interpreting 119 trios. *Genet Med,* 2015, vol. 17, 774-781